# EUROPÄISCHE PATENTSCHRIFT

(11) **EP 1 781 588 B1**
(45) Veröffentlichungstag und Bekanntmachung des Hinweises auf die Patenterteilung: **12.03.2008**
(21) Anmeldenummer: 05787140.2
(22) Anmeldetag: 23.08.2005
(51) Int. Cl.: C07C 41/06, C07C 29/36, C07C 209/60, C07C 67/04

(54) **VERFAHREN ZUR HERSTELLUNG VON 2,7- OCTADIENYLDERIVATEN**
METHOD FOR THE PRODUCTION OF 2,7-OCTADIENYL DERIVATIVES
PROCEDE POUR PRODUIRE DES DERIVES DE 2,7- OCTADIENYLE

(30) Priorität: 28.08.2004 DE 102004041778; 01.08.2005 DE 102005036039
(43) Veröffentlichungstag der Anmeldung: 09.05.2007
(73) Patentinhaber: Evonik Oxeno GmbH, 45772 Marl (DE)
(72) Erfinder: NIERLICH, Franz, 45768 Marl (DE); BORGMANN, Cornelia, 60486 Frankfurt (DE); RÖTTGER, Dirk, 45657 Recklinghausen (DE); HOUBRECHTS, Stephan, 2570 Duffel (BE); MASCHMEYER, Dietrich, 45657 Recklinghausen (DE)
(74) Vertreter: Hirsch, Hans-Ludwig
(86) Internationale Anmeldenummer: PCT/EP2005/054135
(87) Internationale Veröffentlichungsnummer: WO 2006/024614

(56) Entgegenhaltungen:
- WO-A-91/09822
- WO-A-98/12160
- DE-A- 10 312 829
- GB-A- 1 066 765

## Beschreibung

Die Erfindung betrifft ein Verfahren zur Herstellung von 1-Octa-2,7-dienylderivaten durch Umsetzung eines 1,3-Butadien-haltigen Kohlenwasserstoffgemischs, insbesondere Crack-C₄, mit Nucleophilen.

Die dabei aus zwei Molen 1,3-Butadien und einem Mol Nucleophil entstehenden Telomerisationsprodukte (ungesättigte Amine, ungesättigte Alkohole und deren Ester und Ether) sind Ausgangsstoff für organische Synthesen. Die sauerstoffhaltigen Derivate sind Vorstufe für die Herstellung von linearen C₈-Alkoholen und C₈-Olefinen, insbesondere 1-Octanol und 1-Octen. 1-Octanol wiederum wird beispielsweise zur Erzeugung von Weichmachern verwendet. 1-Octen ist ein begehrtes Comonomer zur Modifizierung von Polyethylen und Polypropylen.

Die Telomerisation von Butadien mit einem Nucleophil zu Octadienylderivaten wird durch Metallkomplexe, insbesondere Palladiumverbindungen, katalysiert.

Beispiele für Telomerisationsreaktionen werden unter anderem beschrieben in E. J. Smutny, J. Am. Chem. Soc. 1967, 89, 6793; S. Takahashi, T. Shibano, N. Hagihara, Tetrahedron Lett. 1967, 2451; EP-A-0 561 779, US 3 499 042, US 3 530 187, GB 1 178 812, NL 6 816 008, GB 1 248 593, US 3 670 029, US 3 670 032, US 3 769 352, US 3 887 627, GB 1 354 507, DE 20 40 708, US 4 142 060, US 4 146 738, US 4 196 135, GB 1 535 718, US 4 104 471, DE 21 61 750 und EP-A-0 218 100.

Als Einsatzstoffe für die Herstellung von Octadienylderivaten können reines 1,3-Butadien oder 1,3-Butadien-haltige Kohlenwasserstoffgemische, wie beispielsweise Crack-C₄, verwendet werden.

1,3-Butadien ist wegen des aufwendigen Abtrennverfahren ein relativ teurer Einsatzstoff. Daher ist es meistens wirtschaftlicher, 1,3-Butadien-haltige Kohlenwasserstoffgemische als Einsatzstoff für die Telomerisation zu wählen. Dies ist möglich, da die meisten Begleitstoffe, wie gesättigte Kohlenwasserstoffe, wie beispielsweise n-Butan oder Isobutan, oder Monoolefine, wie beispielsweise Isobuten und lineare Butene, sich in der Telomerisationsreaktion inert verhalten. Nur Inhibitoren, also Stoffe, die die Raum-Zeit-Ausbeute oder die Selektivität mindern oder den Katalysatorverbrauch erhöhen, müssen abgetrennt werden.

Nach DE 195 23 335 ist es ratsam, bei Einsatz der C₄-Fraktion aus Naphthacrackern als 1,3-Butadien-haltigen Rohstoff die Konzentration von acetylenischen Verbindungen und von Allenen im Edukt für die Telomerisation zu begrenzen. Die Summe an acetylenisch und allenisch ungesättigten Verbindungen soll 1 Massen-% bezogen auf 1,3-Butadien nicht überschreiten. Für die Entfernung dieser Störkomponenten wird auf bekannte Verfahren verwiesen, ohne dass bestimmte Verfahren angeführt oder zitiert werden.

Mit Berufung auf diese Patentschrift (DE 195 23 335) weisen DE 101 49 348, DE 102 29 290 und DE 103 29 042 ohne Angabe von Konzentrationsgrenzen darauf hin, dass es günstig sei, acetylenische und allenische Verbindungen vor der Telomerisation zu entfernen.

In WO 91-09822 wird beschrieben, dass es zweckmäßig ist, aus der beim Crackprozess von Naphtha, Gasöl oder LPG erhaltenen C₄-Mischung vor der Telomerisation acetylenischungesättigte Verbindungen durch Selektivhydrierung zu entfernen, soweit diese vorhanden sind. Das dabei verwendete Hydrierverfahren ist dabei nicht offengelegt. In den Beispielen wird ein Rohstoff mit unter 60 ppm Gesamtgehalt an Acetylenen eingesetzt, der keinen ausgewiesenen Anteil an Allenen enthält.

Die Abtrennung der acetylenischen Verbindungen kann durch Extraktion oder Hydrierung dieser Verbindungen erfolgen. Bei der Entfernung der acetylenischen Verbindungen (Methylacetylen (Propin), Ethylacetylen (Butin), Vinylacetylen (Butenin)) durch Hydrierung werden Verfahren angewandt, bei denen mit hoher Selektivität die acetylenischen Verbindungen im Wesentlichen ohne Hydrierung von 1,3-Butadien und Monoolefinen hydriert werden. Als Katalysatoren werden Hydrierkontakte verwendet die Kupfer, Kupfer in Kombination mit unedlen Metallen, Kupfer in Kombination mit Edelmetallen oder Metallkatalysatoren von Metallen der VIII. Nebengruppe des Periodensystems der Elemente, beispielsweise Palladium-Kontakte, verwendet. Entsprechende Verfahren sind unter anderem in folgenden Patentschriften beschrieben: US 6 576 588, US 6 417 419, US 6 225 515, US 6 015 933, US 6 194 626, US 6 040 489, US 4 493 906, US 4 440 956, US 4 101 451, US 3 912 789, US 3 751 508, US 3 541 178, US 3 327 013, US 3 218 268, EP 1 217 060, EP 1 151 790, EP1 070 695, EP 0 273 900, NL 6 613 942.

Die selektive Entfernung von Allenen, insbesondere 1,2-Butadien, durch Hydrierung ist wesentlich schwieriger als die selektive Entfernung von acetylenischen Verbindungen. Die Reaktivität des 1,2-Butadiens bei der Hydrierung ist nur geringfügig höher als die des 1,3-Butadiens. Daher sind bei der Entfernung von 1,2-Butadien aus 1,3-Butadien-haltigen Kohlenwasserstoffgemischen durch Hydrierung 1,3-Butadienverluste unvermeidbar.

Beispielsweise wird in WO 98/12160 ein Verfahren zur gleichzeitigen Entfernung von acetylenischen Verbindungen und 1,2-Butadien aus einem 1,3-Butadien-haltigen Kohlenwasserstoffstrom durch Hydrierung an einem Palladiumkontakt in einer Reaktivdestillationskolonne dargelegt. Obwohl in dem dort angegebenen Beispiel 1 im Kopfprodukt der Gehalt an acetylenischen Verbindungen nur um ca. 60 % und der an 1,2-Butadiens nur um 32 % reduziert wurden, waren bereits 3 % des 1,3-Butadiens durch Hydrierung verloren.

Bei der Herstellung von 2,7-Octadienylderivaten aus Crack-C₄ verliert man entweder einen Teil des 1,3-Butadiens bei der Abtrennung von Inhibitoren (und)/oder man erhält bedingt durch die Inhibitoren eine geringere Raum-Zeit-Ausbeute oder Selektivität bei der Telomerisation oder hat einen höheren Katalysatorverbrauch.

Es bestand daher die Aufgabe, ausgehend von Crack-C₄, ein Verfahren zu entwickeln, dass sich durch hohe Ausbeute an 2,7-Octadienylderivaten bezogen auf 1,3-Butadien im Crack-C₄ und/oder geringen Verbrauch an Telomerisationskatalysator auszeichnet.

Überraschenderweise wurde nun gefunden, dass bei der Telomerisation von 1,3-Butadien zu 2,7-Octadienylderivaten acetylenisch ungesättigte Verbindungen als Inhibitoren wirken, allenisch ungesättigte Verbindungen (beispielsweise 1,2-Butadien) aber keine Wirkung als Inhibitor zeigen. Dies war nicht zu erwarten, da im Stand der Technik, wie z. B. DE 195 23 335 darauf hingewiesen wird, dass die Konzentration an Allenen (wie z. B. 1,2-Butadien) möglichst reduziert werden soll.

Gegenstand der Erfindung ist demnach ein Verfahren zur Herstellung einer Verbindung gemäß der Formel I 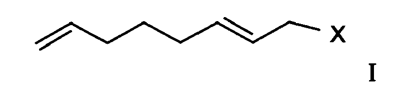 in der X ein Rest OR^{1a} oder NR^{1a}R^{1b} mit R^{1a} und R^{1b} gleich Wasserstoff, einem substituierten oder unsubstituierten Alkyl-, Aryl- oder Acylrest ist, aus einem 1,3-Butadien-haltigen Kohlenwasserstoffstrom, der allenisch ungesättigte Verbindungen sowie mehr als 100 Massen-ppm acetylenisch ungesättigte Verbindungen aufweist, wobei in einem ersten Verfahrensschritt die acetylenisch ungesättigten Verbindungen entfernt werden und in einem zweiten Verfahrensschritt 1,3-Butadien in Gegenwart einer Metallverbindung mit einer aktiven Wasserstoff enthaltenen Verbindung bzw. mit einem Nucleophil (Telogen) umgesetzt wird (Telomerisationsschritt), welches dadurch gekennzeichnet ist, dass der Kohlenwasserstoffstrom, der aus dem ersten Verfahrensschritt erhalten wird und als Edukt in der zweiten Verfahrensstufe eingesetzt wird, einen Gehalt an acetylenisch ungesättigten Verbindungen von kleiner-gleich als 100 Massen-ppm und einen Gehalt an allenisch ungesättigten Verbindungen aufweist, der mindestens 75 % (relativ) des ursprünglichen Gehalts an allenisch ungesättigten Verbindungen beträgt.

Die Verbindung der Formel I kann sowohl in der cis- als auch in der trans-Form vorliegen.

Dadurch, dass im ersten Verfahrensschritt ein Entfernen der Allene unterbleibt, wird zudem erreicht, dass weniger 1,3-Butadien im ersten Verfahrensschritt unbeabsichtigt hydriert oder teilhydriert wird, so dass Ausbeuteverluste in Bezug auf den 1,3-Butadiengehalt im Einsatz-Kohlenwasserstoffstrom minimiert werden können.

Das erfindungsgemäße Verfahren hat außerdem den Vorteil, dass die Allene bzw. Kumulene, also kumulierte Doppelbindungen aufweisenden Verbindungen, wie 1,2-Butadien, die einen wichtigen Ausgangsstoff für die organische Synthese darstellen, in den beiden Verfahrensschritten nicht zerstört werden, sondern im Kohlenwasserstoffstrom vorhanden bleiben und nach dem zweiten Verfahrensschritt, der Telomerisation, bei der Aufarbeitung des Telomerisationsproduktes abgetrennt werden können.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung.

Das erfindungsgemäße Verfahren zur Herstellung einer Verbindung gemäß der Formel I 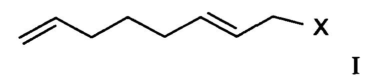 in der X ein Rest OR^{1a} oder NR^{1a}R^{1b} ist, wobei R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R^{1a} und R^{1b} über kovalente Bindungen miteinander verknüpft sein können, aus einem 1,3-Butadien-haltigen Kohlenwasserstoffstrom, der allenisch ungesättigte Verbindungen sowie mehr als 100 Massen-ppm acetylenisch ungesättigte Verbindungen aufweist, wobei in einem ersten Verfahrensschritt die acetylenisch ungesättigten Verbindungen entfernt werden und in einem zweiten Verfahrensschritt 1,3-Butadien in Gegenwart einer Metallverbindung mit einem Nucleophil (einer aktiven Wasserstoff enthaltenen Verbindung, dem Telogen) umgesetzt wird (Telomerisationschritt), zeichnet sich dadurch aus, dass dass der Kohlenwasserstoffstrom, der aus dem ersten Verfahrensschritt erhalten wird und als Edukt in der zweiten Verfahrensstufe eingesetzt wird, einen Gehalt an acetylenisch ungesättigten Verbindungen von kleiner-gleich als 100 Massen-ppm und einen Gehalt an allenisch ungesättigten Verbindungen aufweist, der mindestens 75 % (relativ) des ursprünglichen Gehalts an allenisch ungesättigten Verbindungen beträgt.

Mit dem erfindungsgemäßen Verfahren können insbesondere Verbindungen der IIa oder IIb, 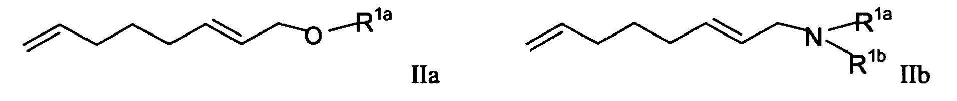 durch Umsetzung von 1,3-Butadien mit einem Nucleophil gemäß den Formeln III, IV oder V

R^{1a}-O-H (III)

(R^{1a})(R^{1b})N-H (IV)

R^{1a}-COOH (V)

hergestellt werden, wobei R^{1a} und R^{1b} die oben genannte Bedeutung haben.

Besonders bevorzugt werden mit dem erfindungsgemäßen Verfahren Verbindungen der Formel I hergestellt, in denen das X für OR^{1a} oder NR^{1a}R^{1b} steht, mit
R^{1a} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl und/oder
R^{1b} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl. Ganz besonders bevorzugt wird mit dem erfindungsgemäßen Verfahren eine Verbindung gemäß der Formel IIa mit R^{1a} = Wasserstoff, Methyl, Ethyl, Phenyl oder Methylcarbonyl hergestellt. Die Verbindungen der Formeln IIa und IIb können sowohl in der cis- als auch in der trans-Form vorliegen.

Ausgangsstoff für das erfindungsgemäße Verfahren sind 1,3-Butadien-reiche Kohlenwasserstoffströme, die unter anderem allenisch ungesättigte Verbindungen und mit einem Anteil von größer 100 Massen-ppm acetylenisch ungesättigte Verbindungen enthalten. Als Kohlenwasserstoffstrom kann insbesondere ein C₄-Kohlenwasserstoffschnitt eingesetzt werden. Die Kohlenwasserstoffströme können vorzugsweise z. B. Mischungen von 1,3-Butadien mit anderen C₄- und C₃- oder C₅-Kohlenwasserstoffen sein. Solche Mischungen fallen beispielsweise bei Spalt(Crack)-Prozessen zur Produktion von Ethylen und Propylen an, in denen Raffineriegase, Naphtha, Gasöl, LPG (liquified petroleum gas), NGL (natural gas liquid) usw. umgesetzt werden. Die bei den Prozessen als Nebenprodukt anfallenden C₄ Schnitte können neben 1,3-Butadien, Monoolefine (1-Buten, cis-But-2-en, trans-But-2-en, Isobuten), gesättigte Kohlenwasserstoffe (n-Butan, Isobutan), acetylenisch ungesättigte Verbindungen (Ethylacetylen (Butin), Vinylacetylen (Butenin), Methylacetylen (Propin)) sowie allenisch ungesättigte Verbindungen (hauptsächlich 1,2-Butadien) enthalten. Weiterhin können diese Schnitte geringe Mengen an C₃- und C₅-Kohlenwasserstoffen enthalten. Die Zusammensetzung der C₄-Schnitte ist abhängig vom jeweiligen Spaltverfahren, den Betriebsparametern und dem Einsatzstoff. Die Konzentrationen der einzelnen Komponenten liegen typischerweise in folgenden Bereichen:

| Komponente | Massen-% |
|---|---|
| 1,3- Butadien | 25 - 70 |
| 1-Buten | 9-25 |
| 2-Butene | 4-20 |
| Isobuten | 10-35 |
| n-Butan | 0,5 - 8 |
| Isobutan | 0,5 - 6 |
| Σ acetylenischer Verbindungen | 0,05 - 4 |
| 1,2-Butadien | 0,05-2 |

Im erfindungsgemäßen Verfahren werden vorzugsweise Kohlenwasserstoffgemische mit einem 1,3-Butadiengehalt von größer 35 Massen-% eingesetzt.

Die Einsatzkohlenwasserstoffe können häufig Spuren von Sauerstoff-, Stickstoff-, Schwefel-, Halogen-, insbesondere Chlor- und Schwermetallverbindungen aufweisen, die im erfindungsgemäßen Verfahren störend sein könnten. Es ist daher zweckmäßig, diese Stoffe zunächst abzutrennen. Störende Verbindungen können z. B. Stabilisatoren, wie z. B. tert.-Butylbrenzcatechin (TBC), oder Kohlendioxid oder Carbonyle, wie z. B. Aceton oder Acetaldehyd sein.

Die Abtrennung dieser Verunreinigungen kann z. B. durch Wäschen, insbesondere mit Wasser oder wässrigen Lösungen, oder über Adsorber erfolgen.

Durch eine Wasserwäsche können hydrophile Komponenten aus dem Kohlenwasserstoffgemisch ganz oder teilweise entfernt werden, beispielsweise Stickstoffkomponenten. Beispiele für Stickstoffkomponenten sind Acetonitril oder N-Methylpyrrolidon (NMP). Auch Sauerstoffverbindungen können zum Teil über eine Wasserwäsche entfernt werden. Die Wasserwäsche kann direkt mit Wasser durchgeführt werden oder aber mit wässrigen Lösungen, die z. B. Salze wie z. B. NaHSO₃ aufweisen können (US 3,682,779, US 3,308,201, US 4,125,568, US 3,336,414 oder US 5,122,236).

Es kann vorteilhaft sein, wenn das Kohlenwasserstoffgemisch nach der Wasserwäsche einen Trocknungsschritt durchläuft. Die Trocknung kann nach den im Stand der Technik bekannten Verfahren durchgeführt werden. Bei vorliegen von gelöstem Wasser kann die Trocknung z. B. unter Verwendung von Molekularsieb als Trocknungsmittel oder durch azeotrope Destillation durchgeführt werden. Freies Wasser kann durch Phasentrennung, z. B. mit einem Koaleszer abgetrennt werden.

Adsorber können eingesetzt werden, um Verunreinigungen im Spurenbereich zu entfernen. Dies kann beispielsweise deshalb vorteilhaft sein, weil im zweiten Prozessschritt Edelmetallkatalysatoren zum Einsatz kommen, die bereits auf Spuren von Verunreinigungen mit deutlicher Aktivitätsabnahme reagieren. Oftmals werden Stickstoff- oder Schwefelverbindungen aber auch TBC über vorgeschaltete Adsorber entfernt. Beispiele für Adsorber sind Aluminiumoxide, Molekularsiebe, Zeolithe, Aktivkohle oder mit Metallen imprägnierte Tonerden (z. B. US 4,571,445 oder WO 02/53685). Adsorber werden von diversen Firmen vertrieben, beispielsweise von der Firma Alcoa unter dem Namen Selexsorb^{®}, von UOP oder von Axens, z. B. mit den Produktserien SAS, MS, AA, TG, TGS oder CMG. Aus dem Kohlenwasserstoffstrom, der zunächst gereinigt worden sein kann, werden im erfindungsgemäßen Verfahren in einem ersten Schritt die acetylenisch ungesättigten Verbindungen bis auf einen Gehalt von kleiner-gleich 100 Massen-ppm, bevorzugt kleiner-gleich 50 Massen-ppm und besonders bevorzugt kleiner-gleich 20 Massen-ppm abgetrennt bzw. entfernt, bevor der Kohlenwasserstoffstrom in dem Telomerisationsschritt eingesetzt wird. Das Abtrennen/Entfernen kann z. B. durch Extraktion oder Hydrierung der acetylenisch ungesättigten Verbindungen erfolgen. Gegebenenfalls vorhandenes Methylacetylen kann auch destillativ entfernt werden.

Die Abtrennung der acetylenischen Verbindungen durch Extraktion ist lange bekannt und ist als Aufarbeitungsschritt ein integraler Bestandteil der meisten Anlagen, die 1,3-Butadien aus Crack-C₄ gewinnen. Ein Verfahren zur extraktiven Abtrennung von acetylenisch ungesättigten Verbindungen aus Crack-C₄, wird beispielsweise in Erdöl und Kohle-Erdgas-Petrochemie vereinigt mit Brennstoffchemie Bd. 34, Heft 8, August 1981, Seite 343 - 346 beschrieben. Bei diesem Verfahren werden in einer ersten Stufe durch Extraktivdestillation mit wasserhaltigem NMP die mehrfach ungesättigten Kohlenwasserstoffe sowie die acetylenisch ungesättigten Verbindungen von den Monoolefinen und gesättigten Kohlenwasserstoffen abgetrennt. Aus dem NMP Extrakt werden die ungesättigten Kohlenwasserstoffe destillativ abgetrennt und aus dem Kohlenwasserstoffdestillat durch eine zweite Extraktivdestillation mit wasserhaltigem NMP die acetylenisch ungesättigten Verbindungen mit 4-C-Atomen abgetrennt. Bei der Aufarbeitung von Crack-C₄ wird durch zwei weitere Destillationen reines 1,3-Butadien abgetrennt, wobei als Nebenprodukte Methylacetylen und 1,2-Butadien anfallen. Im Rahmen des erfindungsgemäßen Verfahrens kann das hier beschriebene mehrstufige Verfahren als erster Verfahrensschritt in gleicher Weise durchgeführt werden, wobei auf die destillative Abtrennung des 1,2-Butadiens verzichtet wird.

Optional kann die Abtrennung von acetylenischen Verbindungen aus einem 1,3-Butadien-haltigen Strom unter Verwendung von einer oder mehreren ionischen Flüssigkeit(en) z. B. als Extraktionsmittel erfolgen.

Die durch Extraktion in dieser ersten Verfahrensstufe erhaltenen, 1,3-Butadien aufweisenden Kohlenwasserstoffströme, die 1,2-Butadien und weniger als 100 Massen-ppm an acetylenischen Verbindungen enthalten, können direkt oder nach einer Aufarbeitung, vorzugsweise direkt als Edukt in der zweiten Verfahrensstufe verwendet werden.

Vorzugsweise erfolgt die Entfernung der acetylenisch ungesättigten Verbindungen aus dem eingesetzten Kohlenwasserstoffstrom durch Hydrierung der acetylenisch ungesättigten Verbindungen. Um die Ausbeuteverluste, vor allen die an 1,3-Butadien und 1,2-Butadien zu vermeiden, muss das Hydrierverfahren sehr selektiv sein, d. h., die Hydrierung von 1,3- oder 1,2-Butadien zu linearen Butenen und die Hydrierung von Butenen zu Butanen muss möglichst weitgehend vermieden werden. Für die selektive Hydrierung von acetylenischen Verbindungen in Gegenwart von Dienen und Monoolefine können z. B. kupferhaltige Katalysatoren verwendet werden. Ebenfalls können Katalysatoren, die ein Edelmetall der VIII. Nebengruppe des Periodensystems der Elemente, insbesondere Palladium aufweisen oder Mischkatalysatoren eingesetzt werden. Besonders bevorzugt werden kupferhaltige Katalysatoren oder Katalysatoren, die sowohl Palladium als auch Kupfer aufweisen, verwendet.

Die Verfahren zur selektiven Hydrierung können ein- oder mehrstufig ausgeführt sein. Wenn die Hydrierung in mehreren Stufen bzw. in mehreren hintereinander geschalteten Reaktoren durchgeführt wird, können in den Reaktoren unterschiedliche Katalysatoren eingesetzt werden (EP 0 273 900). Ein weiteres geeignetes zweistufiges Hydrierverfahren beschreibt auch US 4,277,313, bei dem der selektiven Hydrierung von einem acetylenisch ungesättigte Verbindungen aufweisenden Strom eine anschließende Extraktivdestillation zur Abtrennung von 1,3-Butadien folgt.

Die Katalysator-Aktivität und -Selektivität kann zusätzlich beeinflusst werden durch Zusatz von geeigneten Lösemitteln/Solventien wie sie z. B. in US 4,587,369, US 6,194,626 und US 6,271,428 verwendet werden. Weiterhin, kann die Hydrierung auch in einer Reaktivdestillation bzw. einer Destillation mit außenliegendem Reaktor durchgeführt werden. Generell kann die Hydrierung in den Flüssigphase oder in den Gasphase durchgeführt werden.

Die selektive Hydrierung als erster Verfahrensschritt kann vorzugsweise bei einem Druck von 0,1 bis 7 MPa, bevorzugt von 0,3 bis 5 MPa durchgeführt werden. Die Temperatur beträgt vorzugsweise von 20 bis 250 °C, bevorzugt von 20 bis 150 °C und besonders bevorzugt von 30 bis 80 °C (z. B. US 4,440,956, US 4,126,645 und US 6,417,419). Bei der Durchführung der Hydrierung in der Flüssigphase ist es wichtig darauf zu achten, dass der Wasserstoff völlig gelöst vorliegt, so dass das Auftreten von Hot Spots und somit unselektiver Hydrierung möglichst vermieden wird (US 3,912,789).

In einer bevorzugten Ausführungsform der ersten Verfahrensstufe wird die Hydrierung in der Flüssigphase an einem Kupfer-haltigen Katalysator durchgeführt. Ein Verfahren zur Hydrierung, welches als erster Verfahrensschritt durchgeführt werden kann, wird beispielsweise in US 3,912,789 und US 6,417,419 beschrieben. Genauere Details zum Katalysator und zu den Verfahrensbedingungen, bei denen die selektive Hydrierung durchgeführt werden kann, können US 3,912,789 entnommen werden, auf die hier ausdrücklich Bezug genommen wird. Bei der Verwendung dieses Verfahrens als ersten Verfahrensschritt des erfindungsgemäßen Verfahrens kann, je nach Zusammensetzung des Einsatzkohlenwasserstoffgemisches, das Produkt aus der ersten Verfahrensstufe einen höheren Gehalt an allenisch ungesättigten Verbindungen aufweisen als das Edukt. Eine Durchführung des ersten Verfahrensschrittes gemäß US 3,912,789 hat also den Vorteil, dass allenisch ungesättigte Verbindungen, insbesondere 1,2-Butadien bei der Hydrierung nicht nur nicht entfernt werden sondern der Gehalt an diesen Verbindungen je nach Zusammensetzung des ursprünglichen Kohlenwasserstoffstroms im Produktstrom sogar gesteigert werden kann.

Der erste Verfahrensschritt des erfindungsgemäßen Verfahrens wird deshalb bevorzugt in Gegenwart eines Katalysators, der im wesentlichen aus einer Mischung von feinverteiltem, metallischem Kupfer und einer geringen Menge eines mehrwertigen Aktivatormetalls geträgert auf Gamma-Aluminiumoxid mit einer Oberfläche von mehr als 10 m²/g, welches 0,1 bis 1,5 Massen-% Na₂O aufweist. Als Aktivatormetalle können die Katalysatoren z. B. Silber, Platin, Palladium, Mangan, Nickel, Kobalt, Chrom und/oder Molybdän aufweisen. Die Katalysatoren können bei nachlassender Aktivität einfach wie in US 3,912,789 beschrieben, z. B. durch Kontaktieren des Katalysators zunächst mit einem sauerstoffhaltigen Gas (Abbrennen) und anschließendes Reduzieren des oxidierten Katalysators mit Wasserstoff regeneriert werden. Bevorzugt weist der erste Verfahrensschritt mindestens zwei Hydrierreaktoren auf, so dass bei Regenerierung eines Katalysators der Gesamtprozess weiter betrieben werden kann.

Der erste Verfahrensschritt selbst kann z. B. so durchgeführt werden, dass der Katalysator in einer Reaktionszone eines Reaktors vorgelegt wird, welche dann vom Kohlenwasserstoffstrom durchströmt wird. Die Reaktion kann bei Temperaturen von 10 bis 150 °C, bevorzugt von 50 bis 100 °C durchgeführt werden. Die Reaktion kann bei jedem Druck durchgeführt werden, wobei vorzugsweise ein solcher Druck gewählt wird, bei dem der Prozessstrom in homogener flüssiger Phase vorliegt. Normalerweise wird der erste Verfahrensschritt bei Überdruck durchgeführt werden. Vorzugsweise wird die Reaktion bei einem Druck von 1,013 bis 2,026 MPa durchgeführt.

Olefinisch ungesättigte Verbindungen aufweisende Kohlenwasserstoffströme, die bis zu mehrere Volumenprozenten an acetylenisch ungesättigten Verbindungen aufweisen, können gemäß dieser Ausführungsart des erfindungsgemäßen ersten Verfahrensschrittes selektiv hydriert werden. Besonders gute Ergebnisse werden erzielt, wenn nicht mehr als 0,2 Volumenprozent an acetylenisch ungesättigten Verbindungen im Kohlenwasserstoffstrom vorhanden sind. Unter solchen Bedingungen kann der Kohlenwasserstoffstrom mit einer Geschwindigkeit von 1 bis 5 1*(1*h)⁻¹ bezogen auf das Katalysatorbettvolumen durch das Katalysatorbett geleitet werden. Weitere Details können wiederum US 3,912,789 entnommen werden.

Das molare Verhältnis von Wasserstoff zu acetylenischer Bindung beträgt vorzugsweise zumindest 1. Besonders bevorzugt wird der erste Verfahrensschritt bei einem molaren Überschuss an Wasserstoff durchgeführt. Bevorzugt wird der erste Verfahrensschritt des erfindungsgemäßen Verfahrens bei einem molaren Verhältnis von Wasserstoff zu acetylenisch ungesättigten Bindungen von 1 bis 2 durchgeführt.

Der erste Verfahrensschritt der selektiven Hydrierung kann z. B. auch gemäß US 6,417,419 durchgeführt werden. Auch bei diesem Verfahren wird ein kupferhaltiger Katalysator eingesetzt. Bei dieser Ausführungsform des erfindungsgemäßen ersten Verfahrenschrittes wird die selektive Hydrierung bevorzugt bei einer Temperatur von 20 bis 80 °C, bevorzugt bei einem Druck von 1,5 bis 5,0 MPa und einer Belastung (LHSV) im Bereich von 0,5 bis 10 durchgeführt. Vorzugsweise wird die selektive Hydrierung unter Zugabe von soviel Wasserstoff durchgeführt, dass das Verhältnis von Wasserstoff zu Acetylenen von 1 bis 5 beträgt.

Ein weiteres als erster Verfahrensschritt einsetzbares Verfahren zur selektiven Hydrierung von acetylenisch ungesättigten Verbindungen beschreiben beispielsweise EP 1 070 695, US 6,225,515, US 6,015,933 und US 6,194,626, insbesondere US 6,040,489, die zum Offenbarungsgehalt der vorliegenden Erfindung gehören. Bei diesem Verfahren wird ein 1,3-Butadien aufweisender Strom zusammen mit Wasserstoff und einem Lösemittel in eine katalytische Extraktivdestillationseinheit geführt, die einen Katalysator aufweist, der zur Hydrierung von acetylenisch ungesättigten Verbindungen geeignet ist. Butane und Butene, die in dem Lösemittel weniger gut löslich sind, werden als Überkopfstrom abdestilliert und aus der Destillationseinheit entfernt. Butadiene und Acetylene, die im Lösemittel besser löslich sind, werden mit dem Lösemittel zur Reaktionszone getragen, die in der katalytischen Extraktivdestillationseinheit vorhanden ist. In der Reaktionszone werden die Acetylene zu den Hydrierprodukten umgewandelt. Hydrierprodukte, die nicht Butadiene sind, werden von den Butadienen durch die in der Einheit stattfindende Extraktivdestillation abgetrennt. Der das Lösemittel und Butadiene aufweisende Strom wird aus der Einheit als Extraktstrom entfernt und einer Stripperkolonne zugeführt, in der das Lösemittel vom Butadien abgetrennt wird. Eine weitere destillative Auftrennung der Butadiene in 1,2- und 1,3-Butadien kann unterbleiben.

Als Katalysatoren können in diesem Verfahren (US 6,040,489) insbesondere die in den oben genannten Schutzrechten eingesetzten Katalysatoren, insbesondere Katalysatorzusammensetzungen, die Kupfer, ein oder mehrere Metalle der VIII. Nebengruppe des Periodensytems der Elemente oder Mischungen davon, mit einem anorganischen oxidischen Trägermaterial aufweisen. Neben diesen Materialien können weitere Aktivatormetalle vorhanden sein. Bevorzugte Katalysatoren weisen eine Zusammensetzung auf, die Kupfer aktiviert mit einen oder mehreren Metallen aus der Gruppe Silber, Platin, Palladium, Mangan, Kobalt, Nickel, Chrom und Molybdän auf eine Aluminiumsupport. Besonders bevorzugte Katalysatoren weisen eine Zusammensetzung auf, die Kupfer, Nickel, Mangan und Kobalt, dispergiert auf gamma-Aluminiumoxid, insbesondere auf Aluminiumoxid, welches eine BET-Oberfläche von 150 bis 250 m²/g aufweist, aufweisen.

Die selektive Hydrierung wird vorzugsweise bei einem molaren Verhältnis von Wasserstoff zu Acetylen von 1 bis 5, bevorzugt bei einem Verhältnis von 1 bis 3, besonders bevorzugt bei einem Verhältnis bis 2 durchgeführt. Die Reaktionszone wird bei diesem Verfahren vorzugsweise mit einer Temperatur von 30 bis 100 °C, bevorzugt von 32 bis 83 °C und besonders bevorzugt von ca. 50 bis ca. 80 °C durchgeführt. Die Temperatur ist dabei abhängig vom Betriebsdruck, der in der katalytischen Extraktivdestillationseinheit vorzugsweise von 0,1379 MPa bis 1,379 MPa, bevorzugt von 0,1379 MPa bis 3,447 MPa beträgt. Die Temperatur an anderen Stellen in der Extraktivdestillationseinheit, insbesondere am Kopf der Einheit kann bis zu 150 °C oder höher erreichen.

Das Lösemittel wird so gewählt, dass es eine höhere Affinität zu ungesättigten Kohlenwasserstoffen aufweist als zu gesättigten Kohlenwasserstoffen. Geeignete Lösemittel sind z. B. Dimethylacetamid, Dimethylformamid, Furfural, N-Methylpyrrolidon, Formylmorpholin, Hexan und Acetonitril.

Die Strippkolonne wird vorzugsweise bei einem Druck von ca. 0,1034 bis ca. 0,3447 MPa und einer Temperatur von ca. 30 bis ca. 200 °C betrieben. Die Butadiene werden als Kopfprodukt erhalten. Die Sumpffraktion, die das Lösemittel aufweist, kann nach einer gegebenenfalls durchgeführten Aufarbeitung in die katalytische Extraktivdestillationseinheit zurückgeführt werden.

Weitere Details bzw. Parameter und Verfahrenvarianten zu diesem als ersten Verfahrensschritt verwendeten Verfahren können US 6,040,489 entnommen werden.

Weitere ähnliche Hydrierverfahren und deren Parameter können EP 1 070 695, US 6,225,515, US 6,015,933 und US 6,194,626 entnommen werden, wobei US 6,417,419 insbesondere weitere Details zur Zusammensetzung der Katalysatoren entnommen werden können.

Es ist bekannt, dass bei den angegebenen Hydrierbedingungen der Kupferkatalysator an Aktivität verliert und regelmäßig regeneriert werden muss. Für die Regenerierung sind verschiedene Verfahren in der Literatur beschrieben (US 3,912,789, US 6,225,515 und US 6,194,626), wobei insbesondere die alternative Verwendung von zwei Reaktoren beschrieben wird. Die erste Verfahrensstufe umfasst daher mindestens zwei Hydrierreaktoren, so dass bei Regenerierung eines Katalysators der Gesamtprozess weiter betrieben werden kann. Wie in EP 1 070 695 und US 6,225,515 beschrieben, kann die Regeneration dadurch erfolgen, dass der Reaktor, der Katalysator enthält, der regeneriert werden soll, bei Katalysatorregenerationsbedingungen mit Wasserstoff und einem Lösemittel in Kontakt gebracht wird. Die Temperatur beträgt dabei von 32 bis 260 °C, der Druck von 1,034 bis 3,447 MPa und einer LHSV des Lösemittels von 0,5 bis 10 h⁻¹. Die Zeit zwischen aufeinanderfolgende Regenerierungen kann zudem verlängert werden durch gleichzeitige Dosierung von Kohlenwasserstoffstrom und Lösemittel während der Hydrierung (EP 1 070 695, US 6,271,428 und US 6,194,626). Zur Regeneration kann der Katalysator auch bei erhöhter Temperatur mit einem Oxidationsmittel behandelt werden, wobei als Oxidationsmittel bevorzugt Sauerstoff, insbesondere Luftsauerstoff eingesetzt wird. Dieses Regenerieren (Abbrennen) von Katalysatoren wird für Kupferkatalysatoren in US 3,912,789 und US 3,897,511 und für Palladiumkatalysatoren in US 4,551,443 beschrieben.

Der erste Verfahrensschritt kann in einem Reaktor oder in mehreren Reaktoren durchgeführt werden, wobei diese in Serie oder parallel geschaltet sein können. Werden im ersten Verfahrensschritt des erfindungsgemäßen Verfahrens mehrere Reaktoren oder Reaktorbetten verwendet, die parallel, in Reihe oder als Kombinationen hiervon betrieben werden, kann die Einspeisung des Wasserstoffs bei jedem Reaktor/-bett an einer oder an mehreren Einspeisepunkten erfolgen (US 4,704,492, US 4,126,645, US 4,704,492 oder US 6,417,419). Die Aufteilung der Gesamtwasserstoftmenge auf verschiedene Einspeisepunkte kann zu einer erhöhten Selektivität der Hydrierung führen. Dieses Konzept ist beispielsweise Gegenstand des Dokumentes US 4,704,492, welches zum Offenbarungsgehalt der vorliegenden Erfindung zählt.

Nicht in der ersten Verfahrensstufe umgesetzter Wasserstoff kann nach der Stufe nach bekannten Verfahren ganz oder teilweise abgetrennt werden oder zusammen mit den C₄-Kohlenwasserstoffen in die zweite Verfahrensstufe eingespeist werden. Die Abtrennung des Wasserstoffs kann beispielsweise in einem Ausgasebehälter oder als Abgasstrom im Rahmen einer Destillation erfolgen.

Um die Verluste an 1,3- und 1,2-Butadien sehr gering zu halten, werden die acetylenisch ungesättigten Verbindungen vorzugsweise nicht vollständig hydriert. Aus diesem Grund enthalten die aus dem ersten Verfahrensschritt erhaltenen Kohlenwasserstoffströme vorzugsweise von 0 bis 1 Massen-ppm, bevorzugt zumindest 0,1 Massen-ppm und besonders bevorzugt zumindest 0,5 Massen-ppm an acetylenisch ungesättigten Verbindungen. Der Grad der Hydrierung kann durch eine geeignete Wahl der Prozessparameter, wie z. B. Auswahl des Katalysators, Verweilzeit des Reaktionsgemisches im Reaktor, Reaktionstemperatur und Menge und/oder Druck des eingesetzten Wasserstoffs eingestellt werden, wobei die geeigneten Parameter durch einfache Vorversuche ermittelt werden können.

Der Gehalt an allenisch ungesättigten Verbindungen (z. B. 1,2-Butadien) im aus dem ersten Verfahrensschritt erhaltenen Kohlenwasserstoffstrom kann, abhängig vom Einsatzstoff, z. B. im Bereich von 0,05 bis 2 Massen-% liegen. Der Gehalt an allenisch ungesättigten Verbindungen (z. B. 1,2-Butadien) im aus dem ersten Verfahrensschritt erhaltenen Kohlenwasserstoffstrom beträgt vorzugsweise zumindest 80 % (relativ), bevorzugt zumindest 85 % (relativ), besonders bevorzugt zumindest 90 % (relativ) und ganz besonders bevorzugt zumindest 95 % (relativ) des ursprünglichen Gehalts an allenisch ungesättigten Verbindungen.

Der 1,3-Butadiengehalt liegt abhängig von der Konzentration im Ausgangskohlenwasserstoffstrom im Bereich der Eingangskonzentration. Durch Hydrierung von Butenin zu 1,3-Butadien kann sich der 1,3-Butadiengehalt entsprechend der vorhandenen Buteninmenge erhöhen. Gleichzeitig kann ein geringer Teil des 1,3-Butadiens durch Hydrierung verloren gehen oder in Nebenreaktionen beispielsweise zu Hochsiedern (Grünöl) umgesetzt werden. Im beanspruchten Verfahren liegt der Butadiengehalt nach der ersten Verfahrensstufe vorzugsweise im Bereich maximal 10 % (absolut) über und minimal 10 % (absolut) unter der 1,3-Butadien-Eingangskonzentration, bevorzugt im Bereich von 5 % (absolut) über und 5 % (absolut) unter der 1,3-Butadien-Eingangskonzentration der ersten Verfahrensstufe.

Der Hydrieraustrag kann eine geringe Menge an Hochsiedern (Grünöl) enthalten, die während der Hydrierung entstanden sind. Es kann vorteilhaft sein, wenn die Hochsieder aus dem als Hydrieraustrag des ersten Verfahrensschritts erhaltenen Kohlenwasserstoffstroms vor der Zuführung zum zweiten Verfahrensschritt (Telomerisation) abgetrennt werden. Die Hochsieder können z. B. aus Verbindungen bestehen, die mehr als 4 oder 5 Kohlenstoffatome aufweisen, wobei die Verbindungen (Grünöl), die während des Prozesses entstehen, hauptsächlich mehr als 5 Kohlenstoffatome aufweisen. Komponenten mit 5 Kohlenstoffatomen werden hauptsächlich als Verunreinigungen des Rohstoffs in den Prozess eingebracht, können aber wahlweise im Rahmen der Abtrennung des Grünöls mit abgetrennt werden oder ganz oder teilweise im C₄-Strom verbleiben. Die Hochsieder können vor der Telomerisation, beispielweise durch Destillation, abgetrennt werden. Vorzugsweise wird der Hydrieraustrag, also das Verfahrensprodukt der ersten Verfahrensstufe direkt, also ohne Abtrennung des Hochsieders, als Edukt im zweiten Verfahrensschritt (Telomerisation) eingesetzt.

Optional können 1,3-Butadienströme aus anderen Quellen mit dem Austrag aus der ersten Verfahrensstufe gemischt werden und dieses Gemisch kann in der Telomerisation eingesetzt werden, wobei die Konzentration an acetylenisch ungesättigten Verbindungen im Gesamtstrom, der der Telomerisation zugeführt wird, kleiner-gleich 100 ppm beträgt. Dies können beispielsweise Ströme sein, die in einer Butadienanlage, in der 1,3-Butadien durch Extraktivdestillation gewonnen wird, anfallen.

Wie bereits oben ausgeführt weist das Kohlenwasserstoffgemisch, das in den Telomerisationsschritt eingespeist wird, bevorzugt einen Gehalt an acetylenisch ungesättigten Verbindungen kleiner-gleich 50 Massen-ppm, besonders bevorzugt von 20 Massen-ppm auf.

Die im erfindungsgemäßen Verfahren im Telomerisationsschritt neben dem Kohlenwasserstoffstrom aus dem ersten Verfahrensschritt eingesetzten Nucleophile sind bevorzugt Verbindungen der Formeln III, IV und V 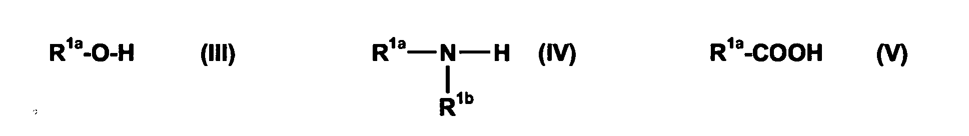 worin R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, - COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, - Cl, -OH, -CF₃, -NO₂ enthalten können, und wobei die Reste R^{1a} und R^{1b} über kovalente Bindungen miteinander verknüpft sein können. Besonders bevorzugt werden als Nucleophile solche Verbindungen eingesetzt, bei denen die Reste R^{1a} und R^{1b} gleich Wasserstoff, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl sind.

Speziell sind dies:
- Wasser, Ammoniak,
- Monoalkohole und Phenole wie zum Beispiel Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol oder 2,7-Octadien-1-ol, Phenol,
- Dialkohole wie zum Beispiel Ethylenglykol, 1,2-Propandiol, 1,3-Propandiol, 1,4-Butandiol, 1,2-Butandiol, 2,3-Butandiol und 1,3-Butandiol,
- Hydroxyverbindungen wie zum Beispiel α-Hydroxyessigsäureester,
- primäre Amine wie zum Beispiel Methylamin, Ethylamin, Propylamin, Butylamin, Octylamin, 2,7-Octadienylamin, Dodecylamin, Ethylendiamin oder Hexamethylendiamin,
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin oder
- Carbonsäuren wie Ameisensäure, Essigsäure, Propansäure, Butensäure, Isobutensäure, Benzoesäure, 1,2 Benzoldicarbonsäure (Phthalsäure).

Ganz besonders bevorzugte werden als Nucleophile im Telomerisationsschritt Methanol, Ethanol, 2-Ethlyhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure, Phenol, Dimethylamin, Methylamin, Ammoniak und/oder Wasser eingesetzt. Vorteilhafterweise wird als Nucleophil Methanol eingesetzt.

Nucleophile, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Für das Verhältnis von Nucleophil zum 1,3-Butadien in der Telomerisationsreaktion ist die Anzahl der aktiven Wasserstoffatome im Telogen zu berücksichtigen. So hat beispielsweise Methanol ein aktives Wasserstoffatom, Ethylenglykol hat zwei, Methylamin hat zwei usw.

Pro Mol aktivem Wasserstoffatom des Nucleophils, das mit dem 1,3-Butadien reagieren kann, werden bevorzugt 0,001 Mol bis 10 Mol 1,3-Butadien in der Telomerisationsreaktion eingesetzt. Bei einer Reaktionsführung mit einer flüssigen Phase wird ein Verhältnis von 0,1 Mol bis 2 Mol 1,3-Butadien pro Mol aktivem Wasserstoff besonders bevorzugt.

Als Katalysator für die Telomerisation werden Komplexe, insbesondere Metallcarbenkomplexe der Metalle Palladium (Pd), Eisen (Fe), Ruthenium (Ru), Osmium (Os), Kobalt (Co), Rhodium (Rh), Iridium (Ir), Nickel (Ni) oder Platin (Pt) eingesetzt. Bevorzugt werden Palladiumverbindungen, insbesondere Palladiumcarbenkomplexe als Katalysator im Telomerisationsschritt verwendet.

Die Liganden in den als Katalysator eingesetzten Metallkomplexen sind beispielweise trivalente Phosporverbindungen oder Carbene. Bevorzugt werden Metallkomplexe als Katalysator verwendet, die mindestens ein durch Heteroatome stabilisiertes Carben als Ligand aufweisen. Beispiele für derartige Liganden werden unter anderem in den Dokumenten DE 101 28 144, DE 101 49 348, DE 101 48 722, DE 100 62 577, EP 1 308 157 und WO 01/66248 beschrieben. Diese Dokumente und insbesondere die dort beschriebenen Liganden zählen zum Offenbarungsgehalt der vorliegenden Anmeldung. Darüber hinaus kann der aktive Komplex noch weitere Liganden tragen.

Geeignete Carben-Liganden sind insbesondere Verbindungen mit den Strukturformeln VI bis IX:

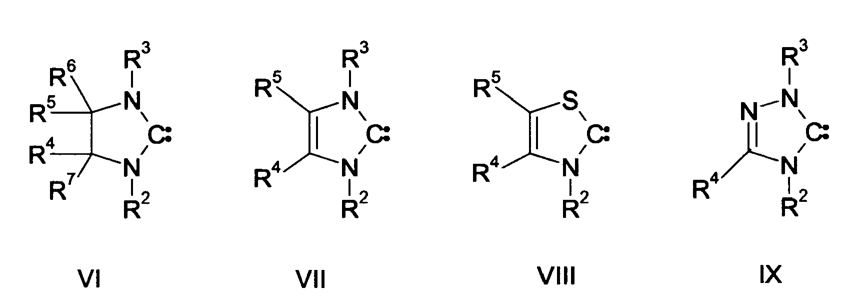

Die Reste R2 bis R7 haben in den Strukturformeln VI bis IX folgende Bedeutung:
- R²; R³:: gleich oder verschieden a) lineare, verzweigte, substituierte oder unsubstituierte cyclische oder alicyclische Alkylgruppen mit 1 bis 24 Kohlenstoffatomen,
oder b) substituierte oder unsubstituierte, mono- oder polycyclische Arylgruppen mit 6 bis 24 Kohlenstoffatomen
oder c) mono- oder polycyclischer, substituierter oder unsubstituierter Heterocyclus mit 4 bis 24 Kohlenstoffatomen und mindestens einem Heteroatom aus der Gruppe N, O, S
- R⁴, R⁵, R⁶, R⁷:: gleich oder verschiedenen
Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Aryl- und Heteroarylgruppen 5 bis 24 Kohlenstoffatome beinhalten und die Reste R⁴ und R⁵ auch Teil eines verbrückenden aliphatischen oder aromatischen Ringes sein können.
R² und R³ stehen insbesondere
- für lineare, verzweigte, cyclische oder alicyclische Alkylgruppen mit 1 bis 24 Kohlenstoffatomen,
- für mono- oder polycyclische Arylgruppen mit 6 bis 24 Kohlenstoffatomen oder
- für einen mono- oder polycyclischen Ring, der mindestes ein Heteroatom ausgewählt aus den Elementen Stickstoff, Sauerstoff und Schwefel enthält,
die gegebenenfalls weitere Substituenten ausgewählt aus den Gruppen -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -Aryl-, -Alkyl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂ aufweisen. Die Gruppen Alkyl der Substituenten weisen 1 bis 24 und die Gruppen Aryl der Substituenten 5 bis 24 Kohlenstoffatome auf.

Die Reste R⁴, R⁵, R⁶ und/oder R⁷ können jeweils gleich oder verschieden sein und mindestens einen Substituenten aus der Gruppe -H, -CN, -COOH, -COO-Alkyl, -COO-Aryl, -OCO-Alkyl, -OCO-Aryl, -OCOO-Alkyl, -OCOO-Aryl, -CHO, -CO-Alkyl, -CO-Aryl, -Aryl, -Alkyl, - Alkenyl, -Allyl, -O-Alkyl, -O-Aryl, -NH₂, -NH(Alkyl), -N(Alkyl)₂, -NH(Aryl), -N(Alkyl)₂, -F, -Cl, -Br, -I, -OH, -CF₃, -NO₂, -Ferrocenyl, -SO₃H, -PO₃H₂ aufweisen, wobei die Alkylgruppen 1 bis 24, bevorzugt 1 bis 20, die Alkenylgruppen 2 bis 24, die Alylgruppen 3 bis 24 und die mono- oder polycyclischen Arylgruppen 5 bis 24 Kohlenstoffatome beinhalten können.

Die Reste R⁴ bis R⁶ können z. B. über (CH₂)- oder (CH)-Gruppen miteinander kovalent verknüpft sein.

Substituenten mit aciden Wasserstoffatomen können an Stelle der Protonen auch Metall- oder Ammoniumionen aufweisen.

Die Reste R² und R³ stehen unter anderem für mono- oder polycyclische Ringe, die mindestens ein Heteroatom enthalten. Dies sind beispielsweise Reste, die sich von fünf- und sechsgliedrigen Heteroalkanen, Heteroalkenen und Heteroaromaten wie 1,4-Dioxan, Morpholin, γ-Pyran, Pyridin, Pyrimidin, Pyrazin, Pyrrol, Furan, Thiophen, Pyrazol, Imidazol, Thiazol und Oxazol ableiten. In der nachfolgenden Tabelle sind konkrete Beispiele für derartige Reste R² und R³ widergegeben. Darin bezeichnet - jeweils den Anknüpfuugspunkt zum Fünfring-Heterozyklus.

| | | | |
|---|---|---|---|
| 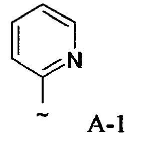 | 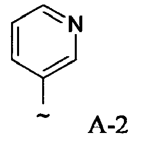 | 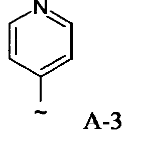 | 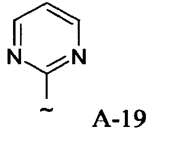 |
| 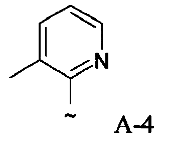 | 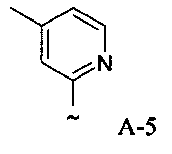 | 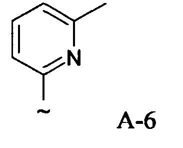 | 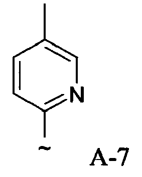 |
| 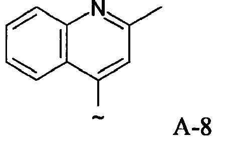 | 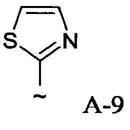 | 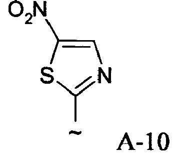 | 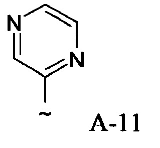 |
| 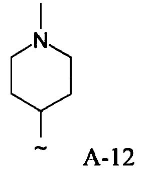 | 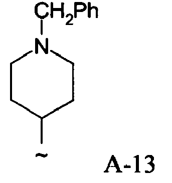 | 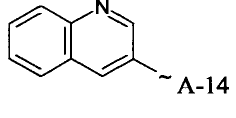 | 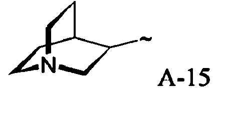 |
| 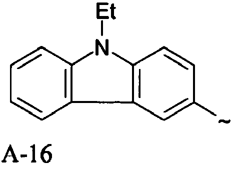 | 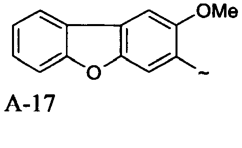 | 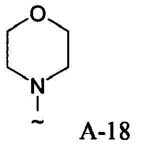 | |

Besonders bevorzugt stehen die Reste R² und R³ für substituierte oder unsubstituierte Phenylreste, beispielsweise 2,4,6-Trimethylphenyl oder 2,6-Diisopropylphenyl. Besonders bevorzugt stehen die Reste R⁴, R⁵, R⁶ und R⁷ für Wasserstoff, Methyl, F oder Cl.

Die Metallcarbenkomplexe können als solche in die Telomerisationsreaktion eingesetzt oder in situ während dieser Reaktion erzeugt werden. Bei der Herstellung des Katalysators im Reaktionsgemisch wird meistens ein quartäres Ammoniumsalz entsprechender Struktur mit einer Base zum Carben umgesetzt, wovon sich zumindest eine Teilmenge an das in der Lösung vorliegende Metall koordiniert.

Als Lösemittel für die Telomerisationsreaktion findet im allgemeinen das eingesetzte Nucleophil Verwendung, wenn es bei Reaktionsbedingungen als Flüssigkeit vorliegt. Es können jedoch auch andere Lösemittel eingesetzt werden. Die eingesetzten Lösemittel sollten dabei weitgehend inert sein. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonsäureester wie zum Beispiel Essigsäureethylester, Ether wie beispielsweise Dipropylether, Diethylether, Dimethylether, Methyloctylether, Methyltertiärbutylether, Ethyltertiärbutylether 3-Methoxyoctan, Dioxan, Tetrahydrofuran, Anisol, Alkyl- und Arylether von Ethylenglycol, Diethylenglycol, Triethylenglycol, Tetraethylenglycol, Polyethylenglycol, Propylenglycol, Dipropylenglycol, Tripropylenglycol und Polypropylenglycol und andere polare Lösemittel wie zum Beispiel Sulfolan, Dimethylsulfoxid, Ethylencarbonat, Propylencarbonat und Wasser. Auch Ionische Flüssigkeiten, beispielsweise Imidazolium- oder Pyridiniumsalze, können als Lösemittel eingesetzt werden. Die Lösemittel können allein oder als Mischungen verschiedener Lösemittel eingesetzt werden.

Die Temperatur, bei der die Telomerisationsreaktion ausgeführt wird, liegt vorzugsweise im Bereich von 10 bis 180 °C, bevorzugt im Bereich von 30 bis 120 °C und besonders bevorzugt im Bereich von 40 bis 100 °C. Der Reaktionsdruck beträgt vorzugsweise von 1 bis 300 bar, bevorzugt von 1 bis 120 bar, besonders bevorzugt von 1 bis 64 bar und ganz besonders bevorzugt von 1 bis 20 bar.

Die Konzentration des Katalysators, formal angegeben in ppm (Masse) an Katalysatormetall bezogen auf die Gesamtmasse, beträgt 0,01 ppm bis 1000 ppm, bevorzugt 0,5 bis 100 ppm, besonders bevorzugt 1 bis 50 ppm.

Das Verhältnis [Mol/Mol] von Carben zu Metall beträgt vorzugsweise von 0,01 : 1 bis 250 : 1, bevorzugt von 1 : 1 bis 100: 1 und besonders bevorzugt von 1 : 1 bis 50: 1. Neben den Carbenliganden können noch weitere Liganden, beispielsweise Phosphorliganden wie Triphenylphosphin, in der Reaktionsmischung vorliegen.

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe Amine, Alkoholate, Phenolate, Alkalimetallsalze, Erdalkalimetallsalze eingesetzt.

Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Alkoholate von Alkali- und/oder Erdalkalielementen, Phosphate, Hydrogenphosphate oder/und Hydroxide bevorzugt von Lithium, Natrium, Kalium, Calcium, Magnesium, Cäsium, Ammonium- und Phosphoniumverbindungen. Bevorzugt sind als Zusatz Hydroxide der Alkali- und Erdalkalielemente und Metallsalze des Nucleophils nach den allgemeinen Formeln III, IV oder V.

Vorzugsweise werden von der basischen Komponente von 0,01 Mol-% bis 10 Mol-% (bezogen auf das Olefin), bevorzugt von 0,1 Mol-% bis 5 Mol-% und ganz besonders bevorzugt von 0,2 Mol-% bis 1 Mol-% eingesetzt.

Die Telomerisation kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die Telomerisation wird, um eine hohe Raum-Zeit-Ausbeute zu erhalten, nicht bis zum vollständigen Umsatz des 1,3-Butadiens durchgeführt. Es ist zweckmäßig, den Umsatz auf maximal 95 %, bevorzugt auf 88 % zu begrenzen.

Der Austrag aus der zweiten Verfahrensstufe kann z. B. hauptsächlich aus dem Telomerisationsprodukt, Nebenprodukten, "inerten C₄-Kohlenwasserstoffen", Restmengen an 1,3-Butadien, Restmengen an Nucleophil und Katalysatorsystem (Katalysatormetall, Liganden, Basen etc.) bzw. dessen Folgeprodukten und gegebenenfalls zugesetzten Lösemitteln bestehen. Auch das 1,2-Butadien ist in diesem Austrag vorhanden.

Die im Austrag der Telomerisation vorhandenen Allene, insbesondere das 1,2-Butadien, können z. B. durch Destillation aus dem Telomerisierungsaustrag abgetrennt werden.

Die Auftrennung des Austrags des zweiten Verfahrensschrittes kann aber auch ganz allgemein nach bekannten technischen Verfahren wie beispielsweise Destillation oder Extraktion erfolgen. Es kann beispielsweise eine destillative Trennung in folgende Fraktionen erfolgen:
- eine C₄-Fraktion, die n-Butan, Isobutan, 1-Buten, 2-Butene, Isobuten, 1,3-Butadien, 1,2-Butadien und gegebenenfalls ganz oder teilweise das Nucleophil enthält,
- eine Fraktion mit dem Zielprodukt (2,7-Octadienylderivat),
- eine Fraktion mit dem Nebenprodukt und/oder
- eine Fraktion mit dem Katalysator und
- gegebenenfalls eine Fraktion mit dem Nucleophil und/oder
- gegebenenfalls eine Lösemittelfraktion

Die Fraktion mit dem Nucleophil, die Fraktion mit dem Lösemittel sowie die Fraktion mit dem Katalysator kann jeweils ganz oder teilweise in die zweite Verfahrensstufe zurückgeführt oder aber einer Aufarbeitung zugeführt werden.

Das Zielprodukt gemäß Formel I wird als solches verwandt oder dient als Vorstufe für andere Stoffe. Beispielsweise kann aus dem Zielprodukt 1-Methoxyoctadi-2,7-en durch Hydrierung der beiden Doppelbindungen und anschließende Methanol-Abspaltung 1-Octen hergestellt werden.

In einer bevorzugten Ausführungsform umfasst das Verfahren einen dritten Verfahrensschritt, in dem die C₄-Fraktion vom restlichen Austrag des zweiten Verfahrensschrittes abgetrennt wird. Die bei der Abtrennung erhaltene Fraktion von C₄-Kohlenwasserstoffen kann dabei noch einen Teil des Nucleophils enthalten, insbesondere dann, wenn sich zwischen Nucleophil einer oder mehrerer der C₄-Komponenten Azeotrope ausbilden. Beispiele für Nucleophile, die derartige Azeotrope bilden, sind Wasser und Methanol.

Die C₄-Fraktion kann auf verschiedene Arten aufgearbeitet werden. Zum einen kann aus der C₄-Fraktion zunächst das 1,2-Butadien, z. B. über Destillation und/oder Extraktivdestillation abgetrennt werden und einer weiteren Verwendung zugeführt werden. Zum anderen kann die C₄-Fraktion einer Selektiv-Hydrierung zugeführt werden, in welcher die Diene entfernt werden, also Reste an 1,3-Butadien und das 1,2-Butadien zu 1-Buten und 2-Butenen umgesetzt werden. Derartige Hydrierungen sind aus dem Stand der Technik bekannt und werden beispielsweise in US 5475173, DE 3119850 und F. Nierlich, F. Obenhaus, Erdöl & Kohle, Erdgas, Petrochemie (1986) 39, 73 - 78 beschrieben. Technisch werden sie sowohl einals auch mehrstufig ausgeführt. Bevorzugt erfolgt die Hydrierung in der Flüssigphase an heterogenen Palladium-Trägerkatalysatoren. Das in der C₄-Fraktion gegebenenfalls vorhandene Nucleophil kann, falls notwenig, vor oder nach der Hydrierung mittels bekannter Verfahren abgetrennt werden. Leicht in Wasser lösliche Nucleophile (beispielsweise Methanol) können z. B. über eine Wasserwäsche entfernt werden. Zur Trocknung des C₄-Stroms haben sich unter anderem Trocknungskolonnen bewährt. Die so erhaltene Mischung der weitgehend 1,3-Butadien-, 1,2-Butadien- und Nucleophil- freien C₄-Kohlenwasserstoffe (Butadiengehalt vorzugsweise kleiner 5000 ppm) entspricht weitestgehend handelsüblichem Raffinat I und kann entsprechend den bekannten Verfahren wie Raffinat I weiterverarbeitet bzw. aufgearbeitet werden. Beispielsweise kann es zur Herstellung von tert.-Butylalkohol, Diisobuten (oder Isooktan), Methyl-tert.-butylether, Ethyl-tert.-butylether, 1-Buten oder C₄-Di- und Oligomeren eingesetzt werden.

Wird als Nucleophil in der zweiten Verfahrensstufe Methanol oder Ethanol verwendet, ergibt sich die Option, das Nucleophil nicht zu entfernen, sondern den Austrag der Hydrierung direkt einer Verätherung zuzuführen, in der der Alkohol mit dem in dem C₄-Strom enthaltenen Isobuten zu Methyl-tert.-Butylether bzw. Ethyl-tert.-Butylether umgesetzt wird. Auch diese Umsetzung erfolgt nach in der Technik bekannten Verfahren, meist unter Katalyse von Ionentauschern. Für einen vollständigen Umsatz des Isobutens muss ggf. zusätzlicher Alkohol zugefügt werden.

Die folgenden Beispiele sollen die Erfindung näher erläutern, ohne den Schutzbereich zu beschränken, der sich aus der Beschreibung und den Patenansprüchen ergibt.

### Beispiel 1: Selektivhydrierung mit einem Kupfer-haltigen Katalysator

Die Hydrieranlage beinhaltete einen gefluteten Tricklebett-Reaktor mit den Dimensionen 14 mm Durchmesser und 2 m Länge und war mit einem äußeren Kreislauf versehen. Der Reaktor wurde elektrisch beheizt, so dass die Reaktion adiabatisch durchgeführt werden konnte. Das Volumen des Katalysators betrug 0,3071. Als Katalysator wurde ein Kupfer-Zink-Katalysator verwendet: 6 % Cu auf ZnO-Tabletten des Typs H9016 von der Firma Degussa. Die Eingangstemperatur betrug 30 °C, der Druck im Reaktor wurde mit Wasserstoff auf 10 bar gedrückt. Die Gesamtzufuhr des C₄-Kohlenwasserstoffgemisches betrug 1,853 kg. Die Durchführung der selektiv Hydrierung erfolgte in Anlehnung an GB 1,066,765. Die Zusammensetzung des Produktes aus der Selektivhydrierung ist der Tabelle 1 zu entnehmen. Es ist deutlich zu erkennen, dass nach 4 Stunden Versuchsdauer der Gehalt an Vinylacetylen und 1-Butin auf null reduziert worden ist, ohne dass sich der Gehalt an 1,3-Butadien und an 1,2-Butadien wesentlich verändert hat.

### Beispiel 2: Telomerisation von 1,3-Butadien-haltigen C₄-Kohlenwasserstoffgemischen mit Methanol

### Allgemeine Arbeitsvorschrift zur Telomerisation

In einem 100-ml-Schlenkrohr wurden unter Schutzgas 55,9 mg (0,18 mmol) Palladiumacetylacetonat und 0,393 g (0,75 mmol) 1,3-Bis(2,4,6-trimethylphenyl)imidazolium-*o*-kresolat-*o*-kresol in 50 g (1,56 mol) Methanol gelöst. In einem 3-Liter-Autoklaven der Firma Büchi wurden 6,72 g (0,06 mol) *o*-Kresol - im Wasserbad auf 40 °C erwärmt - und 3,47 g (0,06 mol) Natriummethanolat in 115 g (3,59 mol) Methanol und in 100 g (0,52 mol) Tripropylenglykol gelöst. Anschließend wurde mit Hilfe einer Gasdruckdose 550 g eines C₄-Kohlenwasserstoffgemisches in den Autoklaven hineingedrückt (Mengenbestimmung durch Massenverlust in der C₄-Vorratsflasche). Der Autoklav wurde unter Rühren auf Reaktionstemperatur erwärmt, die palladiumhaltige Lösung wurde in einem zum Autoklaven-Inhalt gegeben und die Reaktion mit Hilfe eines online-Gaschromatographen verfolgt. Die Reaktionsdauer betrug 14 h.

### GC-Analytik:

**GC** (1. Säule: DB-WAX/Al₂O₃, 2. Säule: DB-Wax/HP-5MS; Anfangstemperatur: 50°C, Maximum-Temperatur: 200 °C, Anfangszeit: 1 min, Equilibrierungszeit: 3 min; Temperaturprogramm: von 50°C mit 15 °C min⁻¹ auf 200 °C, Laufzeit: 11 min; Inj.: 220 °C, konst. Fluß). t_{R}(C4-Kohlenwasserstoffe) = 2.762 min, t_{R}(Methanol) = 3.152 min, t_{R}(1,7-Octadien) = 3.866 min, t_{R}(trans-1,6-Octadien) = 3.958 min, t_{R}(cis-1,6-Octadien) = 4.030 min, t_{R}(cis-1,3,7-Octatrien) = 4.291 min, t_{R}(trans-1,3,7-Octatrien) = 4.292 min, t_{R}(Vinylcyclohexen) = 4.448 min, t_{R}(i-Butan) = 4.552 min, t_{R}(n-Butan) = 4.822 min, t_{R}(3-MODE) = 5.523 min, t_{R}(trans-Buten) = 6.116 min, t_{R}(1-Buten) = 6.240 min, t_{R}(i-Buten) = 6.412 min, t_{R}(cis-Buten) = 6.616 min, t_{R}(1-MODE) = 6.650 min, t_{R}(1,2-Butadien) = 6.900 min, t_{R}(1,3-Butadien) = 7.526 min. 2,7-Octadienyl-1-methylether (= 1-MODE) 1,7-Octadien-3-methylether (= 3-MODE)

### Beispiel 2.1:

In dem erfindungsgemäßen Beispiel wurde das C₄-Kohlenwasserstoffgemisch aus Beispiel 1 eingesetzt.

### Beispiel 2.2:

In dem erfindungsgemäßen Beispiel wurde ein Acetylen-freies und Allen-haltiges C₄-Kohlenwasserstoffgemisch mit 43,53 Gew.-% 1,3-Butadien, 1,95 Gew.-% i-Butan, 4,79 Gew.-% n-Butan, 4,58 Gew.-% trans-Buten, 17,20 Gew.-% 1-Buten, 24,55 Gew.-% i-Buten, 3,15 Gew.-% cis-Buten, 0,1 Gew.-% 1,2-Butadien eingesetzt.

### Vergleichsbeispiel 2.3:

In dem Vergleichsbeispiel wird ein Acetylen- und Allen-freies C₄-Kohlenwasserstoffgemisch mit 43,19 Gew.-% 1,3-Butadien, 1,73 Gew.-% i-Butan, 6,86 Gew.-% n-Butan, 5,12 Gew.-% trans-Buten, 14,80 Gew.-% 1-Buten, 24,56 Gew.-% i-Buten, 3,57 Gew.-% cis-Buten eingesetzt.

### Vergleichsbeispiel 2.4:

In dem Vergleichsbeispiel wurde ein Acetylen-haltiges und Allen-freies C₄-Kohlenwasserstoffgemisch mit 43,19 Gew.-% 1,3-Butadien, 1,73 Gew.-% i-Butan, 6,86 Gew.-% n-Butan, 5,12 Gew.-% trans-Buten, 14,80 Gew.-% 1-Buten, 24,56 Gew.-% i-Buten, 3,57 Gew.-% cis-Buten, 0,0015 Gew.-% Vinylacetylen und 0,0012 Gew.-% 1-Butin eingesetzt.

### Vergleichsbeispiel 2.5:

In diesem Vergleichsbeispiel wurde ein Acetylen-haltiges und Allen-haltiges C₄-Kohlenwasserstoffgemisch mit 43,53 Gew.-% 1,3-Butadien, 1,95 Gew.-% i-Butan, 4,79 Gew.-% n-Butan, 4,58 Gew.-% trans-Buten, 17,20 Gew.-% 1-Buten, 24,55 Gew.-% i-Buten, 3,15 Gew.-% cis-Buten, 0,11 Gew.-% 1,2-Butadien, 0,0017 Gew.-% Vinylacetylen und 0,0010 Gew.-% 1-Butin eingesetzt.

Die Ergebnisse der Beispiele 2.1 bis 2.5 können Tabelle 2 entnommen werden. Das Vergleichsbeispiel 2.3 zeigt, dass Allene keinen Einfluss auf die Katalyseperformance hat. Die Vergleichsbeispiele 2.4 und 2.5 zeigen ebenfalls, dass Allene keinen Einfluss auf die Katalyse haben. Die Katalyse setzt zwar etwas verlangsamt ein, aber es werden trotzdem 100%ige Umsätze bei gleich hohen Selektivitäten erreicht.

**Tabelle 1: Ergebnis der Selektivhydrierung von C₄-Kohlenwasserstoffgemischen**

| **Zeit** | **i-Butan** | **n-Butan** | **trans-Buten** | **1-Buten** | **i-Buten** | **cis-Buten** | **1,2-Butadien** | **1,3-Butadien** | **Vinylacetylen** | **1-Butin** | **Rest** |
|---|---|---|---|---|---|---|---|---|---|---|---|
| [h] | [Gew.-%] | [Gew.-%] | [Gew.-%] | [Gew.-%] | [Gew.-%] | [Gew.-%] | [Gew.-%] | [Gew.-%] | [Gew.-%] | [Gew.-%] | [Gew.-%] |
| 0,00 | 3,82 | 6,67 | 3,90 | 14,80 | 24,01 | 2,55 | 0,12 | 43,25 | 0,74 | 0,13 | 0,01 |
| 0,25 | 3,81 | 6,52 | 4,03 | 14,81 | 24,02 | 2,59 | 0,12 | 43,33 | 0,62 | 0,12 | 0,04 |
| 0,50 | 3,80 | 6,52 | 4,00 | 14,85 | 23,94 | 2,62 | 0,10 | 43,45 | 0,57 | 0,13 | 0,02 |
| 0,75 | 3,81 | 6,49 | 4,01 | 14,82 | 23,97 | 2,64 | 0,12 | 43,44 | 0,55 | 0,13 | 0,03 |
| 1,00 | 3,78 | 6,51 | 4,05 | 14,88 | 23,97 | 2,60 | 0,14 | 43,41 | 0,52 | 0,12 | 0,04 |
| 1,50 | 3,79 | 6,54 | 4,13 | 15,06 | 24,03 | 2,66 | 0,10 | 43,23 | 0,35 | 0,11 | 0,02 |
| 2,25 | 3,82 | 6,53 | 4,16 | 15,23 | 24,15 | 2,68 | 0,14 | 42,99 | 0,19 | 0,09 | 0,03 |
| 2,50 | 3,82 | 6,52 | 4,16 | 15,32 | 24,01 | 2,64 | 0,14 | 43,14 | 0,12 | 0,11 | 0,03 |
| 3,00 | 3,83 | 6,56 | 4,25 | 15,50 | 24,17 | 2,71 | 0,12 | 42,69 | 0,08 | 0,07 | 0,04 |
| 3,50 | 3,80 | 6,53 | 4,35 | 15,62 | 24,04 | 2,73 | 0,13 | 42,73 | 0,00 | 0,06 | 0,02 |
| 4,00 | 3,85 | 6,53 | 4,43 | 15,96 | 24,21 | 2,70 | 0,11 | 42,19 | 0,00 | 0,00 | 0,02 |
| 4,50 | 3,83 | 6,57 | 4,59 | 16,34 | 24,10 | 2,74 | 0,11 | 41,68 | 0,00 | 0,00 | 0,04 |

**Tabelle 2: Ergebnis der Telomerisation (Beispiel 2)**

| Beispiel 2.1 | | | Beispiel 2.2 | | | Vergleichsbeispiel 2.3 | | | Vergleichsbeispiel 2.4 | | | Vergleichsbeispiel 2.5 | | |
|---|---|---|---|---|---|---|---|---|---|---|---|---|---|---|
| Zeit | Umsatz | Selektivität | Zeit | Umsatz | Selektivität | Zeit | Umsatz | Selektivität | Zeit | Umsatz | Selektivität | Zeit | Umsatz | Selektivität |
| [min] | [%] | [%] | [min] | [%] | [%] | [min] | [%] | [%] | [min] | [%] | [%] | [min] | [%] | [%] |
| 0 | 1,9 | 90,1 | 0 | 2,6 | 100,0 | 0 | 0,0 | 0,0 | 0 | 1,5 | 88,8 | 0 | 3,6 | 100 |
| 140 | 68,4 | 96,6 | 140 | 73,0 | 96,8 | 140 | 65,6 | 96,7 | 138 | 32,5 | 96,4 | 140 | 55,7 | 97,0 |
| 360 | 100,0 | 96,6 | 340 | 100,0 | 96,9 | 830 | 100,0 | 95,9 | 540 | 100,0 | 96,6 | 420 | 100,0 | 96,8 |
| 780 | 100,0 | 96,0 | 820 | 100,0 | 96,3 | 950 | 99,9 | 95,9 | 840 | 100,0 | 96,3 | 840 | 100,0 | 96,1 |

| | | | | | | | | | | | | | | |
|---|---|---|---|---|---|---|---|---|---|---|---|---|---|---|
| Umsatz = Umsatz von 1,3-Butadien, Selektivität = Selektivität zu 1-MODE | | | | | | | | | | | | | | |

## Patentansprüche

1. Verfahren zur Herstellung einer Verbindung gemäß der Formel I 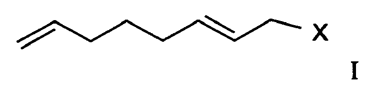 in der X ein Rest OR^{1a} oder NR^{1a}R^{1b} ist, wobei R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, -COO-Alkyl-(C₁-C₈), -CO-Alkyl-(C₁-C₈), -Aryl-(C₃-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R^{1a} und R^{1b} über kovalente Bindungen miteinander verknüpft sein können, aus einem 1,3-Butadien-haltigen Kohlenwasserstoffstrom, der allenisch ungesättigte Verbindungen, sowie mehr als 100 Massen-ppm acetylenisch ungesättigte Verbindungen aufweist, wobei in einem ersten Verfahrensschritt die acetylenisch ungesättigten Verbindungen entfernt werden und in einem zweiten Verfahrensschritt 1,3-Butadien in Gegenwart einer Metallverbindung mit einem Nucleophil umgesetzt wird (Telomerisationsschritt),
**dadurch gekennzeichnet,**
**dass** der Kohlenwasserstoffstrom, der aus dem ersten Verfahrensschritt erhalten wird und als Edukt in der zweiten Verfahrensstufe eingesetzt wird, einen Gehalt an acetylenisch ungesättigten Verbindungen von kleiner-gleich als 100 Massen-ppm und einen Gehalt an allenisch ungesättigten Verbindungen aufweist, der zumindest 75 % des ursprünglichen Gehalts an allenisch ungesättigten Verbindungen beträgt.

2. Verfahren nach Anspruch 1,
**dadurch gekennzeichnet,**
**dass** eine Verbindung gemäß der Formeln IIa oder IIb, 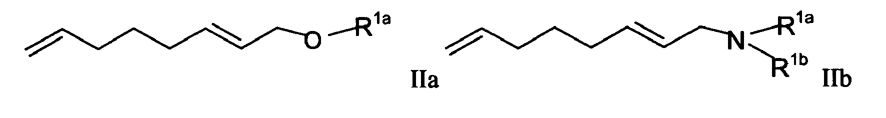 durch Umsetzung von 1,3-Butadien mit einem Nucleophil gemäß den Formeln III, IV oder V
R^{1a}-O-H (III) (R^{1a})(R^{1b})N-H (IV) R^{1a}-COOH (V)
hergestellt wird, wobei R^{1a} und R^{1b} die in Anspruch 1 genannte Bedeutung haben.

3. Verfahren nach Anspruch 1 oder 2,
**dadurch gekennzeichnet,**
**dass** als Kohlenwasserstoffstrom ein C₄-Kohlenwasserstoffschnitt eingesetzt wird

4. Verfahren nach zumindest einem der Ansprüche 1 bis 3,
**dadurch gekennzeichnet,**
**dass** die acetylenisch ungesättigten Verbindungen durch Extraktion abgetrennt werden.

5. Verfahren nach zumindest einem der Ansprüche 1 bis 3,
**dadurch gekennzeichnet,**
**dass** acetylenisch ungesättigten Verbindungen durch Hydrierung entfernt werden.

6. Verfahren nach Anspruch 5,
**dadurch gekennzeichnet,**
**dass** bei der Hydrierung ein kupferhaltiger Katalysator verwendet wird.

7. Verfahren nach Anspruch 5,
**dadurch gekennzeichnet,**
**dass** bei der Hydrierung ein palladiumhaltiger Katalysator verwendet wird.

8. Verfahren nach zumindest einem der Ansprüche 5 bis 7,
**dadurch gekennzeichnet,**
**dass** aus dem als Hydrieraustrag des ersten Verfahrensschritts erhaltenen Kohlenwasserstoffstroms vor der Zuführung zum zweiten Verfahrensschritt (Telomerisation) Verbindungen abgetrennt werden, die mehr als 4 oder 5 Kohlenstoffatome aufweisen.

9. Verfahren nach zumindest einem der Ansprüche 1 bis 7.
**dadurch gekennzeichnet,**
**dass** der als Verfahrensprodukt des ersten Verfahrensschritts erhaltene Kohlenwasserstoffstrom direkt als Edukt im zweiten Verfahrensschritt eingesetzt wird.

10. Verfahren nach zumindest einem der Ansprüche 1 bis 8,
**dadurch gekennzeichnet,**
**dass** der als Verfahrensprodukt des ersten Verfahrensschritts erhaltene Kohlenwasserstoffstrom mit einem 1,3-Butadienstrom aus einer Butadienanlage gemischt wird und dieses Gemisch im zweiten Verfahrensschritt eingesetzt wird.

11. Verfahren nach zumindest einem der Ansprüche 1 bis 10,
**dadurch gekennzeichnet,**
**dass** in dem Kohlenwasserstoffgemisch, das in den Telomerisationsschritt eingespeist wird, der Gehalt an acetylenisch ungesättigten Verbindungen kleiner 50 Massen-ppm beträgt.

12. Verfahren nach Anspruch 11,
**dadurch gekennzeichnet,**
**dass** in dem Kohlenwasserstoffgemisch, das in die Telomerisationsschritt eingespeist wird, der Gehalt an acetylenisch ungesättigten Verbindungen kleiner 20 Massen-ppm beträgt.

13. Verfahren nach einem der Ansprüche 1 bis 12,
**dadurch gekennzeichnet,**
**dass** als Nucleophil im zweiten Verfahrensschritt Methanol, Ethanol, 2-Ethlyhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure, Phenol, Dimethylamin, Methylamin, Ammoniak oder Wasser eingesetzt wird.

14. Verfahren nach einem der Ansprüche 1 bis 13,
**dadurch gekennzeichnet,**
**dass** ein Metallcarbenkomplex als Telomerisationskatalysator in dem zweiten Verfahrensschritt eingesetzt wird.

15. Verfahren nach Anspruch 14,
**dadurch gekennzeichnet,**
**dass** ein Palladiumcarbenkomplex als Telomerisationskatalysator in dem zweiten Verfahrensschritt eingesetzt wird.

16. Verfahren nach einem der Ansprüche 1 bis 15,
**dadurch gekennzeichnet,**
**dass** die allenisch ungesättigten Verbindungen durch Destillation aus dem Telomerisierungsaustrag abgetrennt werden.

17. Verfahren nach einem der Ansprüche 1 bis 15,
**dadurch gekennzeichnet,**
**dass** aus dem Austrag des zweiten Verfahrenschritts eine C₄-Fraktion abgetrennt wird, aus welcher Diene durch Selektivhydrierung entfernt werden.

18. Verfahren nach Anspruch 17,
**dadurch gekennzeichnet,**
**dass** die hydrierte C₄-Fraktion wie Raffinat I aufgearbeitet wird.

19. Verfahren nach zumindest einem der Ansprüche 1 bis 18,
**dadurch gekennzeichnet,**
**dass** X in Formel I für OR^{1a} oder NR^{1a}R^{1b} steht, mit
R^{1a} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Methylphenyl, Naphtyl, 2,4-Di-tert.-butylphenyl, 2,6-Di-tert.-butylmethylphenyl, Hydrogencarbonyl, Methylcarbonyl, Ethlycarbonyl, Propylcarbonyl oder Phenylcarbonyl und
R^{1b} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Kresyl, Naphtyl, 2,4-Di-tert.-butylphenyl, 2,6-Di-tert.-butylmethylphenyl, Hydrogencarbonyl, Methylcarbonyl, Ethlycarbonyl, Propylcarbonyl oder Phenylcarbonyl.

## Claims

1. Process for preparing a compound of the formula I 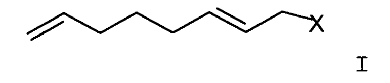 in which X is an OR^{1a} or NR^{1a}R^{1b} radical, where R^{1a} and R^{1b} are selected independently from among hydrogen, a linear, branched or cyclic C₁-C₂₂-alkyl group, an alkenyl group, an alkynyl group, a C₅-C₁₈-aryl group or a -CO-alkyl-(C₁-C₈) group or a -CO-aryl-(C₅-C₁₀) group, with these groups being able to contain substituents selected from the group consisting of -CN, -COOH, -COO-alkyl-(C₁-C₈), -CO-alkyl-(C₁-C₈), -aryl-(C₅-C₁₀), -COO-aryl-(C₆-C₁₀), -CO-aryl- (C₆-C₁₀), -O-alkyl- (C₁-C₈), -O-CO-alkyl-(C₁-C₈), -N-alkyl₂-(C₁-C₈), -CHO, -SO₃H, -NH₂, -F, -Cl, -OH, -CF₃, -NO₂ and the radicals R^{1a} and R^{1b} being able to be joined to one another via covalent bonds, from a 1,3-butadiene-containing hydrocarbon stream comprising allenically unsaturated compounds and more than 100 ppm by mass of acetylenically unsaturated compounds, in which process the acetylenically unsaturated compounds are removed in a first process step and 1,3-butadiene is reacted with a nucleophile in the presence of a metal compound in a second process step (telomerization step), **characterized in that** the hydrocarbon stream which is obtained from the first process step and is used as starting material in the second process step has a content of acetylenically unsaturated compounds of less than or equal to 100 ppm by mass and a content of allenically unsaturated compounds which is at least 75% (relative) of the original content of allenically unsaturated compounds.

2. Process according to Claim 1, **characterized in that** a compound of the formula IIA or IIb, 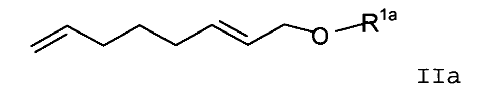 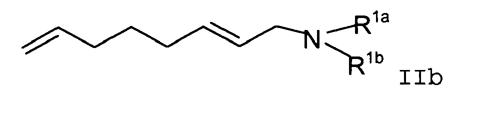 is prepared by reacting 1,3-butadiene with a nucleophile of the formula III, IV or V
R^{1a}-O-H (III) (R^{1a}) ( R^{1b}) N-H (IV) R^{1a}-COOH (V)
where R^{1a} and R^{1b} are as defined in claim 1.

3. Process according to Claim 1 or 2, **characterized in that** a C₄-hydrocarbon fraction is used as hydrocarbon stream.

4. Process according to at least one of Claims 1 to 3, **characterized in that** the acetylenically unsaturated compounds are separated off by extraction.

5. Process according to at least one of Claims 1 to 3, **characterized in that** acetylenically unsaturated compounds are removed by hydrogenation.

6. Process according to Claim 5, **characterized in that** a copper-containing catalyst is used in the hydrogenation.

7. Process according to Claim 5, **characterized in that** a palladium-containing catalyst is used in the hydrogenation.

8. Process according to at least one of Claims 5 to 7, **characterized in that** compounds having more than 4 or 5 carbon atoms are separated off from the hydrocarbon stream obtained as hydrogenation product mixture from the first process step before it is fed to the second process step (telomerization).

9. Process according to at least one of Claims 1 to 7, **characterized in that** the hydrocarbon stream obtained as process product from the first process step is used directly as starting material in the second process step.

10. Process according to at least one of Claims 1 to 8, **characterized in that** the hydrocarbon stream obtained as process product from the first process step is mixed with a 1,3-butadiene stream from a butadiene plant and this mixture is used in the second process step.

11. Process according to at least one of Claims 1 to 10, **characterized in that** the content of acetylenically unsaturated compounds in the hydrocarbon mixture which is fed to the telomerization step is less than 50 ppm by mass.

12. Process according to Claim 11, **characterized in that** the content of acetylenically unsaturated compounds in the hydrocarbon mixture which is fed to the telomerization step is less than 20 ppm by mass.

13. Process according to any of Claims 1 to 12, **characterized in that** the nucleophile used in the second process step is methanol, ethanol, 2-ethylhexanol, octanol, octenol, octadienol, isopropanol, n-propanol, isobutanol, n-butanol, isononanol, formic acid, acetic acid, propionic acid, n-butanoic acid, iso-butanoic acid, benzoic acid, phthalic acid, phenol, dimethylamine, methylamine, ammonia or water.

14. Process according to any of Claims 1 to 13, **characterized in that** a metal-carbene complex is used as telomerization catalyst in the second process step.

15. Process according to Claim 14, **characterized in that** a palladium-carbene complex is used as telomerization catalyst in the second process step.

16. Process according to any of Claims 1 to 15, **characterized in that** the allenically unsaturated compounds are separated off from the telomerization product mixture by distillation.

17. Process according to any of Claims 1 to 15, **characterized in that** a C₄ fraction is separated off from the output from the second process step and dienes are removed from this by selective hydrogenation.

18. Process according to Claim 17, **characterized in that** the hydrogenated C₄ fraction is worked up like raffinate I.

19. Process according to at least one of Claims 1 to 18, **characterized in that** X in the formula I is OR^{1a} or NR^{1a}R^{1b}, where
R^{1a} is H, methyl, ethyl, n-propyl, isopropyl, tert-butyl, n-butyl, sec-butyl, pentyl, hexyl, heptyl, octyl, octenyl, octadienyl, isononyl, 2-ethylhexyl, n-nonyl, phenyl, m-, o- or p-methylphenyl, naphthyl, 2,4-di-tert-butylphenyl, 2,6-di-tert-butylmethylphenyl, hydrogencarbonyl, methylcarbonyl, ethylcarbonyl, propylcarbonyl or phenylcarbonyl and
R^{1b} is H, methyl, ethyl, n-propyl, isopropyl, tert-butyl, n-butyl, sec-butyl, pentyl, hexyl, heptyl, octyl, octenyl, octadienyl, isononyl, 2-ethylhexyl, n-nonyl, phenyl, m-, o- or p-creyl, naphthyl, 2,4-di-tert-butylphenyl, 2,6-di-tert-butylmethylphenyl, hydrogencarbonyl, methylcarbonyl, ethylcarbonyl, propylcarbonyl or phenylcarbonyl.

## Revendications

1. Procédé pour la préparation d'un composé selon la formule I 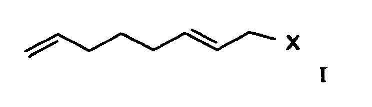 dans laquelle X est un radical OR^{1a} ou NR^{1a}R^{1b}, dans lesquels R^{1a} et R^{1b}, indépendamment l'un de l'autre, sont choisis parmi l'hydrogène, un groupe alkyle linéaire, ramifié ou cyclique en C₁ à C₂₂, un groupe alcényle, un groupe alcynyle, un groupe aryle en C₅ à C₁₈ ou un groupe -CO-alkyl-(C₁-C₈)- ou un groupe -CO-aryl- (C₅-C₁₀)-, sachant que ces groupes peuvent contenir des substituants choisis parmi le groupe -CN, -COOH, -COO-alkyle-(C₁-C₈), -CO-alkyle-(C₁-C₈), -aryle-(C₅-C₁₀), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, -NO₂, et sachant que les radicaux R^{1a} et R^{1b} peuvent être enchaînés ensemble par des liaisons covalentes, à partir d'un courant d'hydrocarbures contenant du 1,3-butadiène, qui comporte des composés alléniquement insaturés, ainsi que plus de 100 ppm en masse de composés acétyléniquement insaturés, sachant que dans une première étape du procédé, les composés acétyléniquement insaturés sont éliminés et que dans une deuxième étape du procédé le 1,3-butadiène est mis en réaction avec un nucléophile (étape de télomérisation) en présence d'un composé métallique,
**caractérisé en ce que**
le courant d'hydrocarbures qui est obtenu à l'issue de la première étape du procédé, et qui est utilisé comme composé de départ dans la deuxième étape du procédé, présente une teneur en composés acétyléniquement insaturés inférieure ou égale à 100 ppm en masse et une teneur en composés alléniquement insaturés qui est égale à au moins 75 % de la teneur initiale en composés alléniquement insaturés.

2. Procédé selon la revendication 1,
**caractérisé en ce**
**qu'**un composé selon les formules IIa ou IIb, 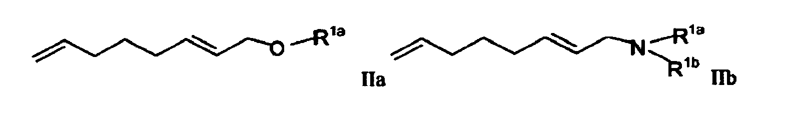 est préparé par réaction de 1,3-butadiène avec un nucléophile selon les formules III, IV ou V
R^{1a}-O-H (III) (R^{1a})(R^{1b})N-H (IV) R^{1a}-COOH (V)
sachant que R^{1a} et R^{1b} ont la signification spécifiée dans la revendication 1.

3. Procédé selon la revendication 1 ou 2,
**caractérisé en ce**
**qu'**un mélange d'hydrocarbures en C₄ est utilisé comme courant d'hydrocarbures.

4. Procédé selon l'une au moins des revendications 1 à 3,
**caractérisé en ce que**
les composés acétyléniquement insaturés sont séparés par extraction.

5. Procédé selon l'une au moins des revendications 1 à 3,
**caractérisé en ce que**
les composés acétyléniquement insaturés sont éliminés par hydrogénation.

6. Procédé selon la revendication 5,
**caractérisé en ce que**,
lors de l'hydrogénation, un catalyseur contenant du cuivre est utilisé.

7. Procédé selon la revendication 5,
**caractérisé en ce que**,
lors de l'hydrogénation, un catalyseur contenant du palladium est utilisé.

8. Procédé selon l'une au moins des revendications 5 à 7,
**caractérisé en ce que**,
avant l'amenée dans la deuxième étape du procédé (télomérisation), les composés qui comportent plus de 4 ou 5 atomes de carbone sont séparés du courant d'hydrocarbures obtenu comme produit de l'hydrogénation de la première étape du procédé.

9. Procédé selon l'une au moins des revendications 1 à 7,
**caractérisé en ce que**,
le courant d'hydrocarbures obtenu comme produit du procédé de la première étape du procédé est utilisé directement comme composé de départ dans la deuxième étape du procédé.

10. Procédé selon l'une au moins des revendications 1 à 8,
**caractérisé en ce que**,
le courant d'hydrocarbures obtenu comme produit du procédé de la première étape du procédé est mélangé à un courant de 1,3-butadiène provenant d'une installation à butadiène et **en ce que** ce mélange est utilisé dans la deuxième étape du procédé.

11. Procédé selon l'une au moins des revendications 1 à 10,
**caractérisé en ce que**,
dans le mélange d'hydrocarbures qui est amené dans l'étape de télomérisation, la teneur en composés acétyléniquement insaturés est de moins de 50 ppm en masse.

12. Procédé selon la revendication 11,
**caractérisé en ce que**,
dans le mélange d'hydrocarbures qui est amené dans l'étape de télomérisation, la teneur en composés acétyléniquement insaturés est de moins de 20 ppm en masse.

13. Procédé selon l'une quelconque des revendications 1 à 12,
**caractérisé en ce que**,
comme nucléophile dans la deuxième étape du procédé, on utilise du méthanol, de l'éthanol, du 2-éthylhexanol, de l'octanol, de l'octénol, de l'octadiénol, de l'isopropanol, du n-propanol, de l'isobutanol, du n-butanol, de l'isononanol, de l'acide formique, de l'acide acétique, de l'acide propionique, de l'acide n-butanoïque, de l'acide iso-butanoïque, de l'acide benzoïque, de l'acide phtalique, du phénol, de la diméthylamine, de la méthylamine, de l'ammoniac ou de l'eau.

14. Procédé selon l'une quelconque des revendications 1 à 13,
**caractérisé en ce**
**qu'**un complexe métal-carbène est utilisé comme catalyseur de télomérisation dans la deuxième étape du procédé.

15. Procédé selon la revendication 14,
**caractérisé en ce**
**qu'**un complexe palladium-carbène est utilisé comme catalyseur de télomérisation dans la deuxième étape du procédé.

16. Procédé selon l'une des revendications 1 à 15,
**caractérisé en ce que**
les composés alléniquement insaturés sont séparés par distillation à partir du produit de la télomérisation.

17. Procédé selon l'une des revendications 1 à 15,
**caractérisé en ce**
**qu'**une fraction en C₄ est séparée du produit de la deuxième étape du procédé, fraction de laquelle les diènes sont éliminés par hydrogénation sélective.

18. Procédé selon la revendication 17,
**caractérisé en ce que**
la fraction en C₄ hydrogénée est traitée comme produit de raffinage I.

19. Procédé selon l'une au moins des revendications 1 à 18,
**caractérisé en ce que**
X représente dans la formule I OR^{1a} ou NR^{1a}R^{1b}, avec R^{1a} égale H, méthyle, éthyle, n-propyle, iso-propyle, tert-butyle, n-butyle, sec-butyle, pentyle, hexyle, heptyle, octyle, octényle, octadiényle, isononyl, 2-éthylhexyl, n-nonyle, phényle, m-, o- ou p-méthylphényle, naphtyle, 2,4-di-tert-butylphényle, 2,6-di-tert-butylméthylphényle, hydrogénocarbonyle, méthylcarbonyle, éthylcarbonyle, propylcarbonyle ou phénylcarbonyle et
R^{1b} égale H, méthyle, éthyle, n-propyle, iso-propyle, tert-butyle, n-butyle, sec-butyle, pentyle, hexyle, heptyle, octyle, octényle, octadiényle, isononyle, 2-éthylhexyl, n-nonyle, phényle, m-, o- ou p-crésyle, naphtyle, 2,4-di-tert-butylphényle, 2,6-di-tert-butylméthylphényle, hydrogénocarbonyle, méthylcarbonyle, éthylcarbonyle, propylcarbonyle ou phénylcarbonyle.
